# EUROPÄISCHE PATENTSCHRIFT

(11) **EP 4 031 893 B1**
(45) Veröffentlichungstag und Bekanntmachung des Hinweises auf die Patenterteilung: **25.12.2024**
(21) Anmeldenummer: 20771810.7
(22) Anmeldetag: 10.09.2020
(51) Int. Cl.: G01R 33/56, A61B 5/055

(54) **ERZEUGUNG VON MRT-AUFNAHMEN DER LEBER**
GENERATION OF MRI IMAGES OF THE LIVER
GÉNÉRATION D'ENREGISTREMENTS IRM DU FOIE

(30) Priorität: 18.09.2019 EP 19197986
(43) Veröffentlichungstag der Anmeldung: 27.07.2022
(73) Patentinhaber: Bayer Aktiengesellschaft, 51373 Leverkusen (DE)
(72) Erfinder: KNOBLOCH, Gesine, 10437 Berlin (DE); LIENERTH, Christian, 60486 Frankfurt/Main (DE)
(74) Vertreter: BIP Patents
(86) Internationale Anmeldenummer: PCT/EP2020/075288
(87) Internationale Veröffentlichungsnummer: WO 2021/052850

(56) Entgegenhaltungen:
- ZHANG XUEJUN ET AL: "Application of an artificial neural network to the computer-aided differentiation of focal liver disease in MR imaging", RADIOLOGICAL PHYSICS AND TECHNOLOGY, SPRINGER JAPAN KK, JP, vol. 2, no. 2, 1 July 2009 (2009-07-01), pages 175 - 182, XP036001157, ISSN: 1865-0333, [retrieved on 20090701], DOI: 10.1007/S12194-009-0062-5
- YEUN-YOON KIM ET AL: "Gadoxetic acid-enhanced magnetic resonance imaging: Hepatocellular carcinoma and mimickers", CLINICAL AND MOLECULAR HEPATOLOGY, vol. 25, no. 3, 21 January 2019 (2019-01-21), Korea, pages 223 - 233, XP055676950, ISSN: 2287-2728, DOI: 10.3350/cmh.2018.0107
- KIM DONG HWAN ET AL: "Arterial subtraction images of gadoxetate-enhanced MRI improve diagnosis of early-stage hepatocellular carcinoma", JOURNAL OF HEPATOLOGY, ELSEVIER, AMSTERDAM, NL, vol. 71, no. 3, 18 May 2019 (2019-05-18), pages 534 - 542, XP085774459, ISSN: 0168-8278, [retrieved on 20190518], DOI: 10.1016/J.JHEP.2019.05.005
- GESINE KNOBLOCH, TIMOTHY COLGAN, XIAOKE WANG, TILMAN SCHUBERT, DIEGO HERNANDO, AND SCOTT REEDER: "Combined Gadoxetic Acid and Gadobenate Dimeglumine Enhanced Liver MRI for Liver Metastasis Detection: A Parameter Optimization Study", INTERNATIONAL SOCIETY FOR MAGNETIC RESONANCE IN MEDICINE, ISMRM, 2030 ADDISON STREET, 7TH FLOOR, BERKELEY, CA 94704 USA, no. 75, 1 June 2018 (2018-06-01), XP040699284
- PETER BANNAS ET AL: "Combined gadoxetic acid and gadofosveset enhanced liver MRI: A feasibility and parameter optimization study : Combined Gadoxetic Acid and Gadofosveset Liver MRI", MAGNETIC RESONANCE IN MEDICINE., vol. 75, no. 1, 3 February 2015 (2015-02-03), US, pages 318 - 328, XP055677040, ISSN: 0740-3194, DOI: 10.1002/mrm.25554
- CANNELLA ROBERTO ET AL: "Common pitfalls when using the Liver Imaging Reporting and Data System (LI-RADS): lessons learned from a multi-year experience", ABDOMINAL RADIOLOGY, SPRINGER US, NEW YORK, vol. 44, no. 1, 2 August 2018 (2018-08-02), pages 43 - 53, XP036812450, ISSN: 2366-004X, [retrieved on 20180802], DOI: 10.1007/S00261-018-1720-Z
- ANJA FISCHER, OLIVER KRAFF, STEPHAN ORZADA, LENA C. SCHÄFER, MARK E. LADD, LALE UMUTLU, AND THOMAS C. LAUENSTEIN: "Ultra-high-field imaging of the biliary tract at 7 Tesla: initial results of Gd-EOB-DTPA-enhanced MRCP", INTERNATIONAL SOCIETY FOR MAGNETIC RESONANCE IN MEDICINE, no. 4022, 1 January 2012 (2012-01-01), XP040626443

## Beschreibung

Die vorliegende Erfindung befasst sich mit der Erzeugung von künstlichen MRT-Aufnahmen der Leber. Gegenstände der vorliegenden Erfindung sind ein Verfahren, ein System und ein Computerprogrammprodukt zur Erzeugung von MRT-Aufnahmen der Leber.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen. Eine Verkürzung der T1-Zeit führt zu einer Zunahme der Signalintensität, eine Verkürzung der T2-Zeit führt zu einer Abnahme der Signalintensität.

Die Wirkung dieser Kontrastmittel ist indirekt, da das Kontrastmittel selbst kein Signal abgibt, sondern nur die Signalintensität in seiner Umgebung beeinflusst.

In T1-gewichteten Aufnahmen führen die paramagnetischen Kontrastmittel zu einer helleren (signalreicheren) Darstellung der Bereiche, die Kontrastmittel beinhalten, gegenüber den Bereichen, die kein Kontrastmittel beinhalten.

In T2-gewichteten Aufnahmen führen superparamagnetisches Kontrastmittel zu einer dunkleren (signalärmeren) Darstellung der Bereiche, die Kontrastmittel beinhalten, gegenüber den Bereichen, die kein Kontrastmittel beinhalten.

Sowohl eine signalreichere als auch eine signalärmere Darstellung führen zu einer Kontrastverstärkung.

Ein Beispiel für ein superparamagnetisches Kontrastmittel sind Eisenoxidnanopartikel (SPIO, engl.: *superparamagnetic iron oxide*)*.*

Beispiele für paramagnetische Kontrastmittel sind Gadolinium-Chelate wie Gadopentetat-Dimeglumin (Handelsname: Magnevist^{®} u.a.), Gadobenat-Dimeglumin (Handelsname: Multihance^{®}), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

Wie beispielsweise von Yeun-Yoon Kim *et al.* (Yeun-Yoon Kim et al.: Gadoxetic acid-enhanced magnetic resonance imaging: Hepatucellular carcinoma and mimickers, Clinical and Molecular Hepatology, 2019, 25: 223-233) und R. Cannella *et al.* (R. Cannella et al.: Common pitfalls when using the Liver Imaging Reporting and Data Sysztem (LIRADS): lessons learned from a multiyear experience, Abdom Radiol, 2019, 44: 43-53) beschrieben, kann Primovist^{®} zur Detektion von Tumoren in der Leber eingesetzt werden. Die Blutversorgung des gesunden Lebergewebes erfolgt in erster Linie über die Pfortader (*Vena portae*), während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Bei der durch Primovist^{®} erzielten Kontrastverstärkung während der Anflutungsphase beobachtet man typische Perfusionsmuster, die Informationen für die Charakterisierung der Läsionen liefern. Die Darstellung der Vaskularisierung hilft, die Läsionstypen zu charakterisieren und den räumlichen Zusammenhang zwischen Tumor und Blutgefäßen zu bestimmen.

Ding Hwan Kim *et al.* offenbaren ein Verfahren zur Diagnose eine hepatozelluläres Karzinoms im Frühstadium auf Basis einer MRT-Aufnahme, die durch Subtraktion einer nativen MRT-Aufnahme von einer MRT-Aufnahme in der arteriellen Phase erzeugt wird (Ding Hwan Kim et al.: Arterial subtraction images of gadocetate-enhanded MRI improve diagnosis of earl-stage hepatocellular carcinoma, J. Hepatol., 2019, 71, 534-542).

Bei T1-gewichteten Bildern führt Primovist^{®} 10-20 Minuten nach der Injektion (in der hepatobiliären Phase) zu einer deutlichen Signalverstärkung im gesunden Leberparenchym, während Läsionen, die keine oder nur wenige Hepatozyten enthalten, z.B. Metastasen oder mittelgradig bis schlecht differenzierte hepatozelluläre Karzinome (HCCs), als dunklere Bereiche auftreten.

Allerdings treten in der hepatobiliären Phase auch die Blutgefäße als dunkle Bereiche auf, so dass in den MRT-Aufnahmen, die während der hepatobiliären Phase erzeugt werden, eine Differenzierung von Leberläsionen und Blutgefäßen allein anhand des Kontrasts nicht möglich ist. Eine Differenzierung zwischen Leberläsionen und Blutgefäßen kann nur im Zusammenhang mit Aufnahmen in der dynamischen Phase erfolgen, bei der die Blutgefäße hervorgehoben werden. Der Radiologe muss sich also bei der Betrachtung von MRT-Aufnahmen, die während der dynamischen Phase erzeugt worden sind, die Blutgefäße merken, um sie in MRT-Aufnahmen, die während der hepatobiliären Phase erzeugt worden sind, von Leberläsionen unterscheiden zu können.

Sowohl P. Bannas *et al.* als auch G. Knobloch *et al.* schlagen vor, zeitversetzt zwei unterschiedliche Kontrastmittel zu applizieren, um Leberläsionen von Blutgefäßen in der hepatobiliären Phase zu differenzieren (P. Bannas et al.: Combined Gadoxetic Acid and Gadofosveset Enhanced Liver MRI: A Feasibility Study and Parameter Optimization, Magnetic Resonance in Medicine, 2016, 75: 318-326; G. Knobloch et al.: Combined Gadoxetic Acid and Gadobenate Dimeglumine Enhanced Liver MRI for Liver Metastasis Detection: A Parameter Optimization Study, International Society for Magnetic Resonance in Medicine, Nr. 75, 2018). Die Applikation von zwei Kontrastmitteln anstelle von einem ist ein zusätzlicher Aufwand, der mit einem zusätzlichen Risiko für den Patienten einhergehen kann. Zudem kann es schwierig sein, für jeden Patienten individuell den richtigen Zeitpunkt für das Verabreichen des zweiten Kontrastmittels zu finden.

Dem Problem der Differenzierung von Leberläsionen von Blutgefäßen in der hepatobiliären Phase widmet sich die vorliegende Erfindung mit den Gegenständen der unabhängigen Patentansprüche 1, 9, 10, 12 und 13.

Bevorzugte Ausführungsformen der vorliegenden Erfindung finden sich in den abhängigen Patentansprüchen, in dieser Beschreibung und in den Zeichnungen.

Ein erster Gegenstand der vorliegenden Erfindung ist ein Verfahren umfassend die Schritte
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein hepatobiliäres Kontrastmittel kontrastverstärkt dargestellt sind,
- Empfangen mindestens einer zweiten MRT-Aufnahme desselben Untersuchungsobjekts, wobei die mindestens eine zweite MRT-Aufnahme dieselbe Leber oder denselben Teil der Leber in der hepatobiliären Phase zeigt,
   wobei gesunde Leberzellen durch das hepatobiliäre oem Kontrastmittel kontrastverstärkt dargestellt sind,
- Erzeugen mindestens einer dritten MRT-Aufnahme durch Kombinieren der mindestens einen ersten MRT-Aufnahme mit der mindestens einen zweiten MRT-Aufnahme und Nivellieren des Kontrastunterschieds zwischen den in der mindestens einen ersten MRT-Aufnahme kontrastverstärkt dargestellten Blutgefäßen und den in der mindestens einen zweiten MRT-Aufnahme kontrastverstärkt dargestellten gesunden Leberzellen,
- Anzeigen und/oder Ausgeben der mindestens einen dritten MRT-Aufnahme und/oder Speichern der mindestens einen dritten MRT-Aufnahme in einem Datenspeicher.

Ein weiterer Gegenstand der vorliegenden Erfindung ist ein System umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts zeigt, wobei Blutgefäße in der Leber durch ein hepatobiliäres Kontrastmittel kontrastverstärkt dargestellt sind,
   - wobei die Steuer- und Recheneinheit konfiguriert ist, die Empfangseinheit zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber in der hepatobiliären Phase zeigt,
      wobei gesunde Leberzellen durch das hepatobiliäre Kontrastmittel kontrastverstärkt dargestellt sind,
   - wobei die Steuer- und Recheneinheit konfiguriert ist, mindestens eine dritte MRT-Aufnahme durch Kombination der mindestens einen ersten MRT-Aufnahme und der mindestens einen zweiten MRT-Aufnahme und Nivellieren des Kontrastunterschieds zwischen den in der mindestens einen ersten MRT-Aufnahme kontrastverstärkt dargestellten Blutgefäßen und den in der mindestens einen zweiten MRT-Aufnahme kontrastverstärkt dargestellten gesunden Leberzellen zu erzeugen,
   - wobei die Steuer- und Recheneinheit konfiguriert ist, die Ausgabeeinheit zu veranlassen, die mindestens eine dritte MRT-Aufnahme anzuzeigen, auszugeben oder in einem Datenspeicher zu speichern.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen mindestens einer ersten MRT-Aufnahme eines Untersuchungsobjekts, wobei die mindestens eine erste MRT-Aufnahme eine Leber oder einen Teil einer Leber des Untersuchungsobjekts zeigt, wobei Blutgefäße in der Leber durch ein hepatobiliäres Kontrastmittel
   kontrastverstärkt dargestellt sind,
- Empfangen mindestens einer zweiten MRT-Aufnahme desselben Untersuchungsobjekts, wobei die mindestens eine zweite MRT-Aufnahme dieselbe Leber oder denselben Teil der Leber in der hepatobiliären Phase zeigt,
   wobei gesunde Leberzellen durch das hepatobiliäre Kontrastmittel kontrastverstärkt dargestellt sind,
- Erzeugen mindestens einer dritten MRT-Aufnahme durch Kombinieren der mindestens einen ersten MRT-Aufnahme mit der mindestens einen zweiten MRT-Aufnahme und Nivellieren des Kontrastunterschieds zwischen den in der mindestens einen ersten MRT-Aufnahme kontrastverstärkt dargestellten Blutgefäßen und den in der mindestens einen zweiten MRT-Aufnahme kontrastverstärkt dargestellten gesunden Leberzellen,
- Anzeigen und/oder Ausgeben der mindestens einen dritten MRT-Aufnahme und/oder Speichern der mindestens einen dritten MRT-Aufnahme in einem Datenspeicher.

Ein weiterer Gegenstand der vorliegenden Erfindung ist die Verwendung eines hepatobiliären Kontrastmittels in einem
MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme, wobei die mindestens eine erste MRT-Aufnahme die Leber oder einen Teil der Leber des Untersuchungsobjekts zeigt, wobei Blutgefäße in der Leber durch das Kontrastmittel kontrastverstärkt dargestellt sind,
- Erzeugen mindestens einer zweiten MRT-Aufnahme, wobei die mindestens eine zweite MRT-Aufnahme dieselbe Leber oder denselben Teil der Leber in der hepatobiliären Phase zeigt, wobei gesunde Leberzellen
   durch das Kontrastmittel kontrastverstärkt dargestellt sind,
- Erzeugen mindestens einer dritten MRT-Aufnahme durch Kombinieren der mindestens einen ersten MRT-Aufnahme mit der mindestens einen zweiten MRT-Aufnahme und Nivellieren des Kontrastunterschieds zwischen den in der mindestens einen ersten MRT-Aufnahme kontrastverstärkt dargestellten Blutgefäßen und den in der mindestens einen zweiten MRT-Aufnahme kontrastverstärkt dargestellten gesunden Leberzellen,
- Anzeigen und/oder Ausgeben der mindestens einen dritten MRT-Aufnahme und/oder Speichern der mindestens einen dritten MRT-Aufnahme in einem Datenspeicher.

Ein weiterer Gegenstand ist ein Kit umfassend ein hepatobiliäres MRT-Kontrastmittel und ein erfindungsgemäßes Computerprogrammprodukt.

Die Erfindung wird nachstehend näher erläutert, ohne zwischen den Erfindungsgegenständen (Verfahren, System, Computerprogrammprodukt, Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die vorliegende Erfindung erzeugt künstliche MRT-Aufnahmen einer Leber oder eines Teils einer Leber eines Untersuchungsobjekts, in denen der Kontrast zwischen den Blutgefäßen in der Leber und den Leberzellen künstlich minimiert ist, um Leberläsionen besser erkennbar werden zu lassen.

Das "Untersuchungsobjekt" ist üblicherweise ein Lebewesen, vorzugsweise ein Säugetier, ganz besonders bevorzugt ein Mensch.

Ein Teil des Untersuchungsobjekts wird einer kontrastverstärkten Magnetresonanztomographie-Untersuchung unterzogen. Der "Untersuchungsbereich", auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden. Der Untersuchungsbereich umfasst zumindest einen Teil der Leber des Untersuchungsobjekts.

Der Untersuchungsbereich wird in ein Grundmagnetfeld eingebracht

Dem Untersuchungsobjekt wird ein Kontrastmittel verabreicht, das sich in dem Untersuchungsbereich verteilt. Das Kontrastmittel wird vorzugsweise intravenös als Bolus verabreicht (z.B. in eine Armvene).

Unter einem "Kontrastmittel" wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Relaxationszeit und/oder der T2- Relaxationszeit.

Erfindungsgemäß ist das Kontrastmittel ein hepatobiliäres Kontrastmittel wie beispielsweise Gd-EOB-DTPA oder Gd-BOPTA.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Der Untersuchungsbereich wird einem MRT-Verfahren unterzogen und dabei werden MRT-Aufnahmen erzeugt (gemessen), die den Untersuchungsbereich während der Untersuchungsphase zeigen.

Die gemessenen MRT-Aufnahmen können als zweidimensionale Bildaufnahmen vorliegen, die eine Schnittebene durch das Untersuchungsobjekt zeigen. Die gemessenen MRT-Aufnahmen können als Stapel zweidimensionaler Bildaufnahme vorliegen, wobei jede einzelne Bildaufnahme des Stapels eine andere Schnittebene zeigt. Die gemessenen MRT-Aufnahmen können als dreidimensionale Aufnahmen (3D-Aufnahmen) vorliegen. Aus Gründen der einfacheren Darstellung wird die Erfindung an einigen Stellen der vorliegenden Beschreibung anhand des Vorliegens von zweidimensionalen MRT-Aufnahmen erläutert, ohne die Erfindung jedoch auf zweidimensionale MRT-Aufnahmen beschränken zu wollen. Dem Fachmann ist klar, wie sich das jeweils Beschriebene auf Stapel zweidimensionaler Bildaufnahmen und auf 3D-Aufnahmen übertragen lässt (siehe hierzu z.B. M. Reisler, W. Semmler: Magnetresonanztomographie, Springer Verlag, 3. Auflage, 2002, ISBN: 978-3-642-63076-7).

Üblicherweise liegen die gemessenen MRT-Aufnahmen als digitale Bilddateien vor. Der Begriff "digital" bedeutet, dass die MRT-Aufnahmen von einer Maschine, in der Regel einem Computersystem, verarbeitet werden können. Unter "Verarbeitung" werden die bekannten Verfahren zur elektronischen Datenverarbeitung (EDV) verstanden.

Digitale Bilddateien können in verschiedenen Formaten vorliegen. Digitale Bilddateien können beispielsweise als Rastergrafiken kodiert werden. Rastergrafiken bestehen aus einer rasterförmigen Anordnung von so genannten Bildpunkten (Pixeln) oder Volumenelement (Voxel), denen jeweils eine Farbe bzw. ein Grauwert zugeordnet ist. Die Hauptmerkmale einer 2D-Rastergrafik sind daher die Bildgröße (Breite und Höhe gemessen in Pixeln, umgangssprachlich auch Bildauflösung genannt) sowie die Farbtiefe. Einem Bildpunkt einer digitalen Bilddatei ist üblicherweise eine Farbe zugeordnet. Die für einen Bildpunkt verwendete Kodierung der Farbe definiert sich unter anderem über den Farbraum und die Farbtiefe. Der einfachste Fall ist ein Binärbild, bei dem ein Bildpunkt einen Schwarzweiß-Wert speichert. Bei einem Bild, dessen Farbe über den so genannten RGB-Farbraum definiert ist (RGB steht für die Grundfarben Rot, Grün und Blau), besteht jeder Bildpunkt aus drei Subpixeln, einem Subpixel für die Farbe Rot, einem Subpixel für die Farbe Grün und einem Subpixel für die Farbe Blau. Die Farbe eines Bildpunktes ergibt durch die Überlagerung (additives Mischen) der Farbwerte der Subpixel. Der Farbwert eines Subpixels ist z.B. in 256 Farbnuancen unterteilt, die Tonwerte genannt werden und üblicherweise von 0 bis 255 reichen. Die Farbnuance "0" eines jeden Farbkanals ist die dunkelste. Haben alle drei Kanäle den Tonwert 0, erscheint der entsprechende Bildpunkt schwarz; haben alle drei Kanäle den Tonwert 255, erscheint der entsprechende Bildpunkt weiß. Bei der Ausführung der vorliegenden Erfindung werden digitale Bilddateien (MRT-Aufnahmen) bestimmten Operationen unterworfen. Die Operationen betreffen dabei überwiegend die Bildpunkte, bzw. die Tonwerte der einzelnen Bildpunkte. Es gibt eine Vielzahl an möglichen digitalen Bildformaten und Farbkodierungen. Vereinfachend wird in dieser Beschreibung davon ausgegangen, dass die vorliegenden Bilder Graustufen-Rastergrafiken mit einer spezifischen Zahl an Bildpunkten sind, wobei jedem Bildpunkt ein Tonwert zugeordnet ist, der den Grauwert des Bildes angibt. Diese Annahme soll jedoch in keiner Weise limitierend verstanden werden. Dem Fachmann der Bildverarbeitung ist klar, wie er die Lehre dieser Beschreibung auf Bilddateien, die in anderen Bildformaten vorliegen und/oder bei denen die Farbwerte anders kodiert sind, übertragen kann.

Nach der intravenösen Applikation eines hepatobiliären Kontrastmittels in Form eines Bolus erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet. Diese Phase beginnt unmittelbar nach der Applikation des Kontrastmittels und dauert üblicherweise 15 bis 60 Sekunden.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "venöse Phase" bezeichnet. Diese Phase kann bereits während der arteriellen Phase beginnen und sich mit dieser überlagern. Üblicherweise startet diese Phase 60 bis 70 Sekunden nach der intravenösen Applikation und dauert üblicherweise 50 bis 70 Sekunden.

An die venöse Phase schließt sich die "Spätphase" an, in der der Kontrast in den Leberarterien weiter absinkt, der Kontrast in den Lebervenen ebenfalls absinkt und der Kontrast in den gesunden Leberzellen allmählich anstiegt. Diese Phase beginnt üblicherweise 100 bis 140 Sekunden nach der Applikation des Kontrastmittels und dauert üblicherweise 50 bis 70 Sekunden.

Die arterielle Phase, die venöse Phase und die Spätphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/jjhep.2019.05.005).

In MRT-Aufnahmen, die den Untersuchungsbereich während der hepatobiliären Phase zeigen, lassen sich Strukturen, die durch Blutgefäße erzeugt werden, oftmals nicht von Strukturen, die durch Leberläsionen hervorgerufen werden, unterscheiden. Daher wird erfindungsgemäß mindestens eine MRT-Aufnahme der Leber oder eines Teils der Leber des Untersuchungsobjekts (künstlich) erzeugt (berechnet), in der die Strukturen, die durch die Blutgefäße verursacht werden, gegenüber dem umgebenden gesunden Lebergewebe unterdrückt sind, damit Leberläsionen besser erkennbar werden.

Dies erfolgt mit Hilfe mindestens einer ersten MRT-Aufnahme und mindestens einer zweiten MRT-Aufnahme.

Als "erste MRT-Aufnahme" wird in dieser Beschreibung eine MRT-Aufnahme bezeichnet, in der Blutgefäße erkennbar sind, die erfindungsgemäß durch ein hepatobiliäres Kontrastmittel kontrastverstärkt dargestellt sind.

Vorzugsweise handelt es sich bei der mindestens einen ersten MRT-Aufnahme um mindestens eine MRT-Aufnahme, die während der dynamischen Phase gemessen worden ist. Besonders bevorzugt handelt es sich um mindestens eine MRT-Aufnahme, die während der arteriellen Phase und/oder der venösen Phase gemessen worden ist. Ganz besonders bevorzugt handelt es sich um eine MRT-Aufnahme, die während der venösen Phase gemessen worden ist. Es ist auch denkbar, dass mindestens zwei "erste MRT-Aufnahmen" verwendet werden, mindestens eine, die in der arteriellen Phase gemessen worden ist und mindestens eine, die in der venösen Phase gemessen worden ist. Vorzugsweise handelt es sich bei der mindestens einen ersten MRT-Aufnahme um eine T1-gewichtete Darstellung.

Bei Verwendung eines paramagnetischen Kontrastmittels sind die Blutgefäße in der mindestens einen ersten MRT-Aufnahme aufgrund der Kontrastverstärkung durch eine hohe Signalintensität gekennzeichnet (signalreiche Darstellung). Diejenigen (zusammenhängenden) Strukturen innerhalb einer ersten MRT-Aufnahme, die eine Signalintensität innerhalb einer empirisch ermittelbaren Bandbreite aufweisen, lassen sich damit Blutgefäßen zuordnen. Damit liegt mit der mindestens einen ersten MRT-Aufnahme eine Information darüber vor, wo in den MRT-Aufnahmen Blutgefäße dargestellt sind bzw. welche Strukturen in den MRT-Aufnahmen auf Blutgefäße (Arterien und/oder Venen) zurückzuführen sind.

Als "zweite MRT-Aufnahme" wird in dieser Beschreibung eine MRT-Aufnahme bezeichnet, die den Untersuchungsbereich während der hepatobiliären Phase zeigt. Während der hepatobiliären Phase wird das gesunde Lebergewebe (Parenchym) kontrastverstärkt dargestellt. Diejenigen (zusammenhängenden) Strukturen innerhalb einer zweiten MRT-Aufnahme, die eine Signalintensität innerhalb einer empirisch ermittelbaren Bandbreite aufweisen, lassen sich damit gesunden Leberzellen zuordnen. Damit liegt mit der mindestens einen zweiten MRT-Aufnahme eine Information darüber vor, wo in den MRT-Aufnahmen gesunde Leberzellen dargestellt sind bzw. welche Strukturen in den MRT-Aufnahmen auf gesunde Leberzellen zurückzuführen sind. Vorzugsweise handelt es sich bei der mindestens einen zweiten MRT-Aufnahme um eine T1-gewichtete Darstellung.

Die Information aus der mindestens einen ersten MRT-Aufnahme über die Blutgefäße wird mit der Information aus der mindestens einen zweiten MRT-Aufnahme über die gesunden Leberzellen kombiniert. Dabei wird mindestens eine dritte MRT-Aufnahme (künstlich) erzeugt (berechnet), bei der der Kontrastunterschied zwischen Strukturen, die auf Blutgefäße zurückzuführen sind und Strukturen, die auf gesunde Leberzellen zurückzuführen sind, nivelliert ist.

Der Begriff "Nivellieren" bedeutet hier "Angleichen" oder "Minimieren". Das Ziel des Nivellierens ist, die Grenzen zwischen Blutgefäßen und gesunden Leberzellen in der mindestens einen dritten MRT-Aufnahme verschwinden zu lassen, und Blutgefäße und gesunde Leberzellen in der mindestens einen dritten MRT-Aufnahme wie ein einheitliches Gewebe erscheinen zu lassen, von dem sich Leberläsionen strukturell durch einen anderen Kontrast abheben
Üblicherweise wird eine (Anzahl = 1) erste MRT-Aufnahme mit einer (Anzahl = 1) zweiten MRT-Aufnahme zu einer (Anzahl = 1) dritten MRT-Aufnahme kombiniert.

Es ist denkbar, dass neben mindestens einen ersten MRT-Aufnahme und mindestens einer zweiten MRT-Aufnahme auch noch mindestens eine native MRT-Aufnahme verwendet wird, um mindestens eine dritte MRT-Aufhahme zu berechnen

Das Erzeugen der dritten (künstlichen) MRT-Aufnahme kann auf verschiedene Weisen erfolgen.

In einer Ausführungsform (Ausführungsform A) der vorliegenden Erfindung umfasst das Erzeugen der mindestens einen dritten MRT-Aufnahme die folgenden Teilschritte:
- Identifizieren von Bereichen in der mindestens einen ersten MRT-Aufnahme, die Blutgefäße darstellen, durch Identifizieren derjenigen Pixel oder Voxel der mindestens einen ersten MRT-Aufnahme, die einen Tonwert aufweisen, der innerhalb eines ersten definierten Tonwert-Bandes liegt, und/oder
- Identifizieren von Bereichen in der mindestens einen zweiten MRT-Aufnahme, die gesunde Leberzellen darstellen, durch Identifizieren derjenigen Pixel oder Voxel der mindestens einen zweiten MRT-Aufnahme, die einen Tonwert aufweisen, der innerhalb eines zweiten definierten Tonwert-Bandes liegt, und
- zumindest partielles Übereinanderlegen der mindestens einen ersten MRT-Aufnahme und der mindestens einen zweiten MRT-Aufnahme und dabei Erzeugen einer dritten MRT-Aufnahme, wobei die Tonwerte der Pixel oder Voxel mit dem Tonwert innerhalb der ersten definierten Bandbreite und der Pixel oder Voxel mit dem Tonwert innerhalb der zweiten definierten Bandbreite auf einen gemeinsamen Tonwert gesetzt werden.

In einer weiteren Ausführungsform (Ausführungsform B) der vorliegenden Erfindung umfasst das Erzeugen der dritten MRT-Aufnahme die folgenden Teilschritte:
- Identifizieren von Blutgefäßen in den ersten MRT-Aufnahmen mittels eines Segmentierverfahrens,
- Identifizieren derjenigen Strukturen in den zweiten MRT-Aufnahmen, die den Blutgefäßen in den ersten MRT-Aufnahmen entsprechen,
- Identifizieren eines (vorzugsweise arithmetisch gemittelten) Tonwerts von gesunden Leberzellen in den zweiten MRT-Aufnahmen,
- Setzen der Tonwerte der Strukturen in den zweiten MRT-Aufnahmen, die den Blutgefäßen in den ersten MRT-Aufnahmen entsprechen, auf den Tonwert von gesunden Leberzellen.

In einer weiteren Ausführungsform (Ausführungsform C) der vorliegenden Erfindung umfasst das Erzeugen der dritten MRT-Aufnahme die folgenden Teilschritte:
- Zuführen der mindestens einen ersten MRT-Aufnahme und der mindestens einen zweiten MRT-Aufnahme einem Vorhersagemodell, wobei das Vorhersagemodell anhand von Referenz-MRT-Aufnahmen mittels überwachten Lernens trainiert worden ist, aus mindestens einer ersten Referenz-MRT-Aufnahme und mindestens einer zweiten Referenz-MRT-Aufnahme mindestens eine dritte Referenz-MRT-Aufnahme zu erzeugen, wobei bei der mindestens einen dritten Referenz-MRT-Aufnahme der Kontrastunterschied zwischen Strukturen, die auf Blutgefäße zurückzuführen sind und Strukturen, die auf gesunde Leberzellen zurückzuführen sind, nivelliert ist,
- Empfangen mindestens einer dritten MRT-Aufnahme als Ausgabe von dem Vorhersagemodell.

Die Ausführungsformen A, B und C werden weiter hinten in der Beschreibung näher erläutert.

Die (künstlich) erzeugte mindestens eine dritte MRT-Aufnahme kann auf einem Monitor angezeigt werden, auf einem Drucker ausgegeben werden und/oder in einem Datenspeicher gespeichert werden. Vorzugsweis wird die mindestens eine dritte MRT-Aufnahme auf einem Monitor angezeigt, wobei Tonwerte der Blutgefäße und/oder Tonwerte der gesunden Leberzellen in der Weise veränderbar sind, dass ihr Wert von dem Wert der mindestens einen ersten MRT-Aufnahme oder der mindestens einen zweiten MRT-Aufnahme kontinuierlich in Richtung ihres Werts in der mindestens einen dritten MRT-Aufnahme veränderbar ist (zum Beispiel mittels eines (virtuellen) Schiebereglers). Diese Ausführungsform hat den Vorteil, dass ein Nutzer zwischen den einzelnen MRT-Aufnahmen hin- und herblenden kann und dabei beispielsweise die Strukturen, die auf Blutgefäße hinweisen, ein- und ausblenden kann, um Läsionen im Lebergewebe erkennen zu können.

Ein Gegenstand der vorliegenden Erfindung ist ein System, mit dem das erfindungsgemäße Verfahren ausgeführt werden kann.

Das System umfasst eine Empfangseinheit, eine Steuer- und Recheneinheit und eine Ausgabeeinheit.

Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Das erfindungsgemäße System ist konfiguriert, mindestens eine erste MRT-Aufnahme und mindestens eine zweite MRT-Aufnahme zu empfangen und diese MRT-Aufnahmen in mindestens einer dritten MRT-Aufnahme miteinander zu kombinieren.

Die Steuer- und Recheneinheit dient der Steuerung der Empfangseinheit, der Koordinierung der Daten- und Signalflüsse zwischen verschiedenen Einheiten und der Prozessierung und Erzeugung von MRT-Aufnahmen. Es ist denkbar, dass mehrere Steuer- und Recheneinheiten vorhanden sind.

Die Empfangseinheit dient zum Empfang von MRT-Aufnahmen. Die MRT-Aufnahmen können beispielsweise von einer Magnetresonanzanlage (zum Beispiel über ein Netzwerk) übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Von der Empfangseinheit werden die mindestens eine erste MRT-Aufnahme und die mindestens eine zweite MRT-Aufnahme und ggf. weitere Daten an die Steuer- und Recheneinheit übermittelt.

Die Steuer- und Recheneinheit ist konfiguriert, mindestens eine dritte MRT-Aufnahme durch Kombination der mindestens einen ersten MRT-Aufnahme und der mindestens einen zweiten MRT-Aufnahme und Nivellieren des Kontrastunterschieds zwischen Blutgefäßen und gesunden Leberzellen zu erzeugen.

Über die Ausgabeeinheit kann die mindestens eine dritte MRT-Aufnahme angezeigt werden (zum Beispiel auf einem Monitor), ausgegeben werden (z.B. über einen Drucker) oder in einem Datenspeicher gespeichert werden.

Die Erfindung wird nachstehend anhand von Figuren näher erläutert, ohne die Erfindung auf die in den Figuren gezeigten Merkmale oder Merkmalskombinationen beschränken zu wollen.

Es zeigen:

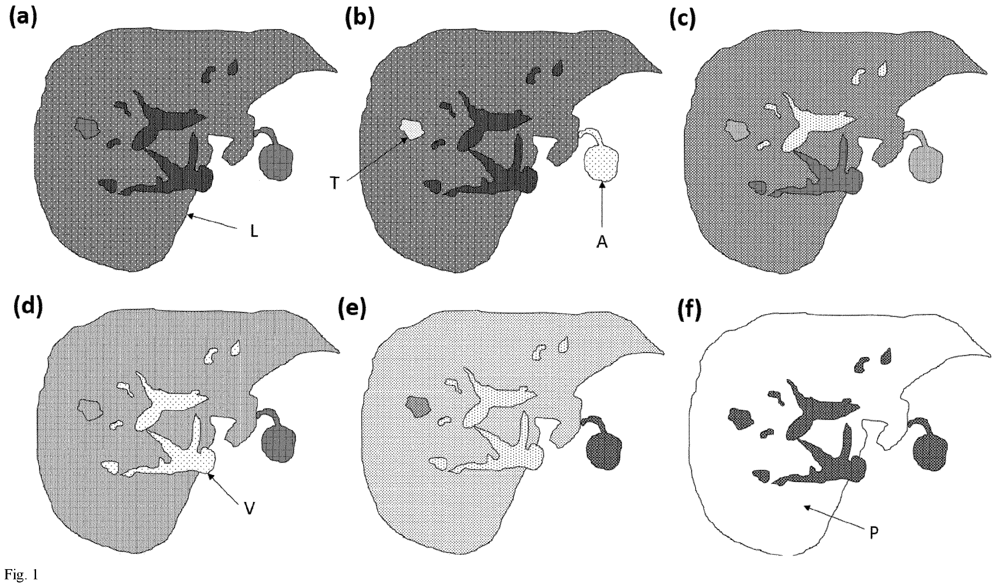

Figur 1 zeigt MRT-Aufnahmen einer Leber während der dynamischen und hepatobiliären Phase. In den Figuren 1 (a), 1 (b), 1 (c), 1 (d), 1 (e) und 1 (f) ist stets derselbe Querschnitt durch die Leber zu unterschiedlichen Zeitpunkten dargestellt. Die in den Figuren 1 (a), 1 (b), 1 (d) und 1 (f) eingezeichneten Bezugszeichen gelten für alle Figuren 1 (a), 1 (b), 1 (c), 1 (d), 1 (e) und 1 (f); sie sind lediglich der besseren Übersicht halber jeweils nur einmal eingezeichnet.
Fig. 1 (a) zeigt den Querschnitt durch die Leber (L) vor der intravenösen Applikation eines hepatobiliären Kontrastmittels. Zu einem Zeitpunkt, der zwischen den Zeitpunkten liegt, die durch die Figuren 1 (a) und 1 (b) dargestellt werden, wurde ein hepatobiliäres Kontrastmittel intravenös (z.B. in eine Armvene) als Bolus verabreicht. Dieses erreicht in Fig. 1 (b) über die Leberarterie (A) die Leber. Dementsprechend wird die Leberarterie signalverstärkt dargestellt (arterielle Phase). Ein Tumor (T), der hauptsächlich über Arterien mit Blut versorgt wird, hebt sich ebenfalls als hellerer (signalverstärkter) Bereich vor dem Leberzellengewebe ab. Zu dem Zeitpunkt, der in Figur 1 (c) dargestellt ist, erreicht das Kontrastmittel die Leber über die Venen. In Figur 1 (d) heben sich die venösen Blutgefäße (V) als helle (signalverstärkte) Bereiche von dem Lebergewebe ab (venöse Phase). Gleichzeitig steigt die Signalintensität in den gesunden Leberzellen, die hauptsächlich über die Venen mit Kontrastmittel versorgt werden, kontinuierlich an (Fig. 1 (c) → 1 (d) → 1 (e) → 1 (f)). In der hepatobiliären Phase, die in Fig. 1 (f) dargestellt ist, sind die Leberzellen (P) signalverstärkt dargestellt; die Blutgefäße und der Tumor weisen kein Kontrastmittel mehr auf und sind entsprechend dunkel dargestellt; die Blutgefäße und der Tumor sind in der MRT-Aufnahme der Fig. 1 (f) nicht unterscheidbar.

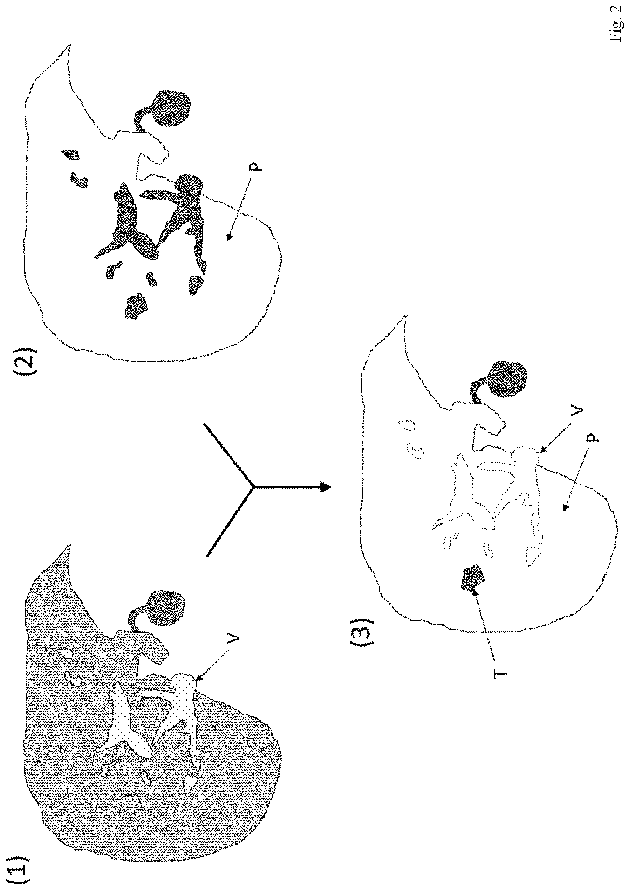

Figur 2 zeigt beispielhaft und schematisch, wie eine erste MRT-Aufnahme (1) und eine zweite MRT-Aufnahme (2) miteinander kombiniert werden, um eine dritte MRT-Aufnahme (3) zu erzeugen. Bei der ersten MRT-Aufnahme handelt es sich um die in Fig. 1 (d) dargestellte MRT-Aufnahme. In ihr sind die venösen Blutgefäße (V) innerhalb der Leber gut zu erkennen. Sie weisen aufgrund des Kontrastmittels eine hohe Signalintensität auf. Bei der zweiten MRT-Aufnahme handelt es sich um die in Fig. 1 (f) dargestellte MRT-Aufnahme. In ihr sind die gesunden Leberzellen (P) gut zu erkennen. Sie weisen aufgrund des Kontrastmittels eine hohe Signalintensität auf. Die erste MRT-Aufnahme (1) und die zweite MRT-Aufnahme (2) sind vorzugsweise gemessene MRT-Aufnahmen. Sie werden miteinander zu der dritten MRT-Aufnahme (3) kombiniert. Die dritte MRT-Aufnahme (3) ist eine künstlich erzeugte, das heißt berechnete MRT-Aufnahme. Dabei können Bereiche innerhalb der MRT-Aufnahme (3) durchaus gemessene Werte beinhalten; die dritte MRT-Aufnahme weist jedoch Bereiche auf, die nicht gemessen, sondern beispielsweise das Ergebnis von Berechnungen sind. In der dritten MRT-Aufnahme (3) ist der Kontrast zwischen den Strukturen, die Blutgewebe darstellen, und den Strukturen, die Leberzellen darstellen, minimiert, so dass der Tumor (T) deutlich erkennbar wird.

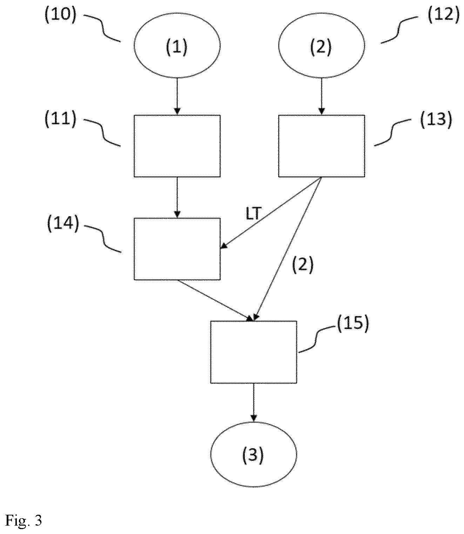

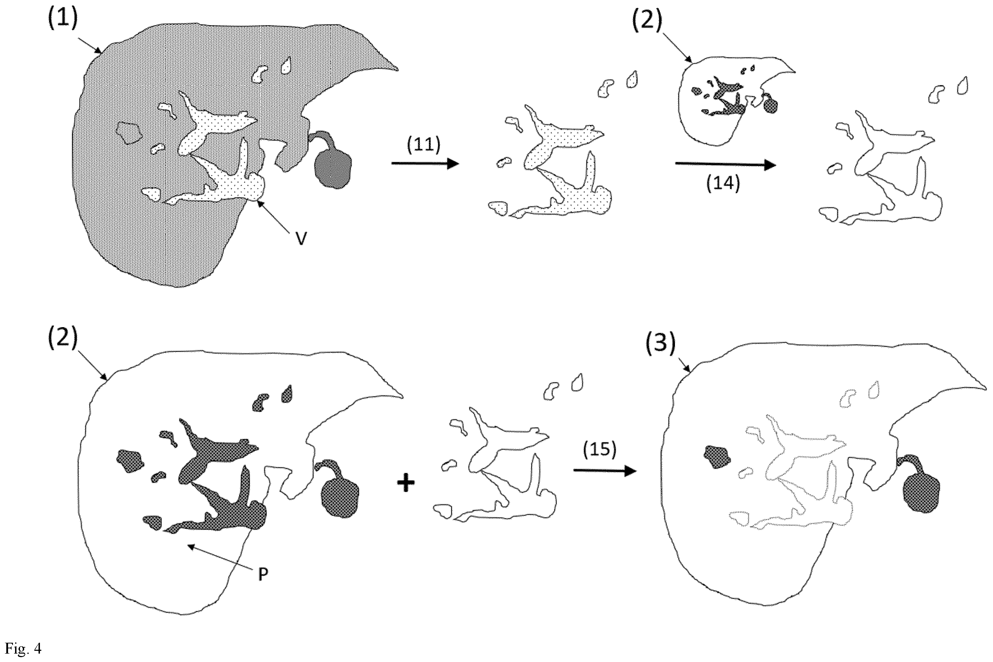

Figur 3 zeigt beispielhaft und schematisch eine Ausführungsform der vorliegenden Erfindung in Form eines Ablaufdiagramms (Ausführungsform A). Dieselbe Ausführungsform ist in Figur 4 anhand von MRT-Aufnahmen dargestellt. Diese Ausführungsform lässt sich auf einzelne zweidimensionale MRT-Aufnahmen anwenden (ist aber nicht auf solche beschränkt).

In einem ersten Schritt (10) wird mindestens eine erste MRT-Aufnahme (1) bereitgestellt, wobei die mindestens eine erste MRT-Aufnahme (1) eine Leber oder einen Teil einer Leber eines Untersuchungsobjekts zeigt, wobei Blutgefäße in der Leber durch ein Kontrastmittel kontrastverstärkt (signalverstärkt) dargestellt sind. Es kann sich dabei beispielsweise um die in Fig. 1 (d) gezeigte MRT-Aufnahme handeln.

Die mindestens eine erste MRT-Aufnahme (1) zeigt venöse Blutgefäße aufgrund des zum Zeitpunkt der Messung der MRT-Aufnahme in den Venen vorhandenen Kontrastmittels im Vergleich zu anderem Gewebe besonders hell (signalverstärkt) an. Diejenigen Bereiche innerhalb der mindestens einen ersten MRT-Aufnahme, die "besonders hell" dargestellt werden, lassen sich also auf venöse Blutgefäße zurückführen.

In einem weiteren Schritt (11) werden diese, in der mindestens einen ersten MRT-Aufnahme "besonders hell" dargestellten Bereiche identifiziert. Dies geschieht vorzugsweise anhand der Tonwerte der Pixel oder Voxel der Bereiche. Liegt der Tonwert eines Pixels oder Voxels oberhalb eines unteren Tonwertschwellenwerts und unterhalb eines oberen Tonwertschwellenwertes, d.h. innerhalb eines definierten ersten Tonwert-Bandes, so kann davon ausgegangen werden, dass das entsprechende Pixel oder Voxel einen Teil eines venösen Blutgefäßes darstellt. Das erste Tonwert-Band (d.h. der untere und der obere Tonwert) lässt sich empirisch gewinnen. Beispielsweise kann ein Radiologe in einer MRT-Aufnahme die Strukturen spezifizieren, die auf Blutgefäße zurückzuführen sind. Die Tonwerte dieser Strukturen können dann das Tonwert-Band definieren. In Schritt (11) werden also diejenigen Bereiche in der mindestens einen ersten MRT-Aufnahme identifiziert, die einen Tonwert innerhalb eines definierten (ersten) Tonwert-Bandes aufweisen.

Denkbar ist auch, dass eine native MRT-Aufnahme von der mindestens einen ersten MRT-Aufnahme abgezogen wird (Differenzbilderstellung). Das Differenzbild zeigt dann lediglich die Strukturen, die durch Kontrastmittel verstärkt werden - und das sind im Fall der mindestens einen ersten MRT-Aufnahme Blutgefäße.

In einem weiteren Schritt (12) wird mindestens eine zweite MRT-Aufnahme (2) bereitgestellt, wobei die mindestens eine zweite MRT-Aufnahme dieselbe Leber oder denselben Teil der Leber zeigt wie die mindestens eine erste MRT-Aufnahme, wobei das gesunde Lebergewebe (Parenchym) durch ein Kontrastmittel kontrastverstärkt dargestellt ist. Es kann sich dabei beispielsweise um die in Fig. 1 (f) gezeigte MRT-Aufnahme handeln.

In der mindestens einen zweiten MRT-Aufnahme können diejenigen Bereiche identifiziert werden, die gesunde Leberzellen darstellen. Dies kann analog zu der Vorgehensweise in Schritt (11) erfolgen, d.h. es werden diejenigen Pixel oder Voxel in der mindestens einen zweiten MRT-Aufnahme identifiziert, deren Tonwert oberhalb eines unteren Tonwertschwellenwerts und unterhalb eines oberen Tonwertschwellenwertes, d.h. innerhalb eines definierten zweiten Tonwert-Bandes liegt. Das zweite Tonwert-Band (d.h. der untere und der obere Tonwert) lässt sich ebenfalls empirisch gewinnen.

Ebenso lässt sich auch hier wieder ein Differenzbild erstellen (zweite MRT-Aufnahme minus native Aufnahme). Das Differenzbild zeigt auch hier lediglich die Strukturen, die durch Kontrastmittel verstärkt werden - und das sind im Fall der mindestens einen zweiten MRT-Aufnahme gesunde Leberzellen.

In einem weiteren Schritt (13) wird ein Tonwert ermittelt. Dieser Tonwert wird hier auch als der Leber-Tonwert bezeichnet. Dabei kann es sich beispielsweise um den arithmetisch gemittelten Tonwert derjenigen Pixel oder Voxel handeln, die in der mindestens einen zweiten MRT-Aufnahme gesundes Lebergewebe darstellen.

In einem weiteren Schritt (14) werden die Tonwerte der Pixel oder Voxel, die in der mindestens einen ersten MRT-Aufnahme venöse Blutgefäße darstellen, auf den Leber-Tonwert gesetzt.

Denkbar ist auch, dass zusätzlich die Tonwerte der Pixel oder Voxel, die in der mindestens einen zweiten MRT-Aufnahme gesunde Leberzellen darstellen, auf den (gemittelten) Leber-Tonwert gesetzt werden.

In einem weiteren Schritt (15) werden die mindestens eine erste MRT-Aufnahme und die mindestens eine zweite MRT-Aufnahme zumindest partiell übereinandergelegt. "Übereinanderlegen" oder auch "Überlagern" bezeichnet die Kombination der mindestens einen ersten MRT-Aufnahme und der mindestens einen zweiten MRT-Aufnahme zu mindestens einer dritten MRT-Aufnahme. Das Übereinanderlegen erfolgt üblicherweise für ein Paar einer ersten MRT-Aufnahme und einer zweiten MRT-Aufnahme Pixel für Pixel bzw. Voxel für Voxel. Hat sich das Untersuchungsobjekt während der Aufnahme der ersten und der zweiten MRT-Aufnahme nicht bewegt, entspricht ein Pixel oder ein Voxel der ersten MRT-Aufnahme genau einem Pixel oder Voxel der zweiten MRT-Aufnahme: die entsprechenden Pixel oder Voxel zeigen denselben Untersuchungsbereich zu unterschiedlichen Zeitpunkten. Zur Erzeugung der dritten MRT-Aufnahme können einige Pixel oder Voxel der ersten MRT-Aufnahme als Pixel oder Voxel der dritten MRT-Aufnahme und einige Pixel oder Voxel der zweiten MRT-Aufnahme als Pixel oder Voxel der dritten MRT-Aufnahme verwendet werden. Es ist ebenso denkbar, dass die Tonwerte einiger Pixel oder Voxel der ersten MRT-Aufnahme mathematisch mit den entsprechenden Pixeln oder Voxeln der zweiten MRT-Aufnahme verknüpft werden: beispielsweise können (normierte) Summen- oder Differenzbilder erzeugt werden. Entscheidend ist, dass der Kontrastunterschied zwischen Bereichen, die auf Blutgefäße zurückzuführen sind, und Bereichen, die auf gesunde Leberzellen zurückzuführen sind, in der dritten MRT-Aufnahme nivelliert wird. Dies kann dadurch geschehen, dass in der dritten MRT-Aufnahme die Tonwerte derjenigen Pixel oder Voxel, die auf Blutgefäße zurückzuführen sind, und die Tonwerte derjenigen Pixel oder Voxel, die auf gesunde Leberzellen zurückzuführen sind, auf denselben Tonwert gesetzt werden, beispielsweise auf den mittleren Leber-Tonwert. Falls sich das Untersuchungsobjekt zwischen der Messung der ersten MRT-Aufnahme und der Messung der zweiten MRT-Aufnahme bewegt hat, muss vor dem Übereinanderlegen der MRT-Aufnahmen eine Bewegungskorrektur vorgenommen werden. Bewegungskorrekturverfahren sind im Stand der Technik beschrieben (siehe zum Beispiel: EP3118644, EP3322997, US20080317315, US20170269182, US20140062481, EP2626718).

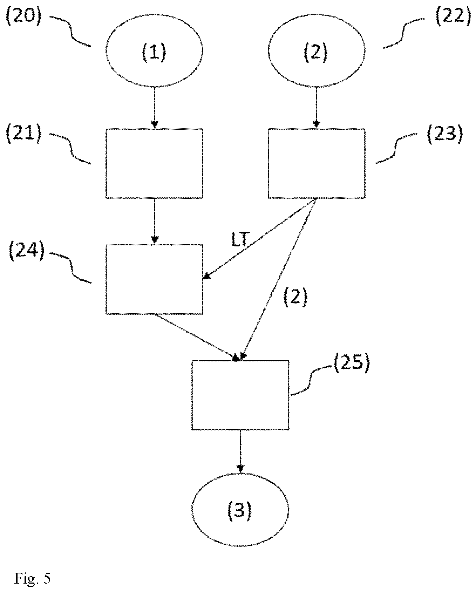

Figur 5 zeigt beispielhaft und schematisch eine weitere Ausführungsform der vorliegenden Erfindung in Form eines Ablaufdiagramms (Ausführungsform B).

In einem ersten Schritt (20) wird eine erste dreidimensionale MRT-Aufnahme (1) bereitgestellt, wobei die mindestens eine erste dreidimensionale MRT-Aufnahme eine Leber oder einen Teil einer Leber eines Untersuchungsobjekts zeigt, wobei Blutgefäße in der Leber durch ein Kontrastmittel kontrastverstärkt dargestellt sind.

In einem weiteren Schritt (21) werden mit Hilfe eines Segmentierverfahrens die Blutgefäße in der ersten dreidimensionalen MRT-Aufnahme identifiziert. Blutgefäße sind in der ersten dreidimensionalen MRT-Aufnahme dadurch charakterisiert, dass sie aufgrund des Kontrastmittels kontrastverstärkt dargestellt sind, also einen Tonwert aufweisen, der innerhalb eines definierten ersten Tonwert-Bandes liegt.

Segmentierverfahren sind vielfältig in der Literatur beschrieben. Beispielhaft seien die folgenden Publikationen aufgeführt: F. Conversano et al.: Hepatic Vessel Segmentation for 3D Planning ofLiver Surgery, Acad Radiol 2011, 18: 461-470; S. Moccia et al.: Blood vessel segmentation algorithms - Review of methods, datasets and evaluation metrics, Computer Methods and Programs in Biomedicine 158 (2018) 71-91; M. Marcan et al.: Segmentation of hepatic vessels from MRI images for planning of electroporation-based treatments in the liver, Radiol Oncol 2014; 48(3): 267-281; T. A. Hope et al.: Improvement of Gadoxetate Arterial Phase Capture With a High Spatio-Temporal Resolution Multiphase Three-Dimensional SPGR-Dixon Sequence, Journal of Magnetic Resonsnce Imaging 38: 938-945 (2013); WO2009/135923A1, US6754376B1, WO2014/162273A1, WO2017/139110A1, WO2007/053676A2, EP2750102A1).

In einem weiteren Schritt (22) wird eine zweite dreidimensionale MRT-Aufnahme (2) bereitgestellt, wobei die zweite dreidimensionale MRT-Aufnahme dieselbe Leber oder denselben Teil der Leber zeigt wie die erste dreidimensionale MRT-Aufnahme, wobei das gesunde Lebergewebe (Parenchym) durch ein Kontrastmittel kontrastverstärkt dargestellt ist.

In einem weiteren Schritt (23) wird ein Tonwert ermittelt. Dieser Tonwert wird hier auch als der Leber-Tonwert bezeichnet. Dabei kann es sich beispielsweise um den arithmetisch gemittelten Tonwert derjenigen Voxel handeln, die in der zweiten dreidimensionalen MRT-Aufnahme gesundes Lebergewebe darstellen.

In einem weiteren Schritt (24) werden die Tonwerte der Voxel, die in der ersten dreidimensionalen MRT-Aufnahme die segmentierten Blutgefäße darstellen, auf den Leber-Tonwert gesetzt.

Denkbar ist, dass auch die Tonwerte der Voxel, die in der zweiten dreidimensionalen MRT-Aufnahme gesunde Leberzellen darstellen, auf den (gemittelten) Leber-Tonwert gesetzt werden.

In einem weiteren Schritt (25) werden die erste dreidimensionale MRT-Aufnahme und die zweite dreidimensionale MRT-Aufnahme in einer dritten dreidimensionalen MRT-Aufnahme miteinander kombiniert. Dazu werden die aus der ersten dreidimensionalen MRT-Aufnahme segmentierten Blutgefäße mit dem Leber-Tonwert in die zweite dreidimensionale MRT-Aufnahme eingefügt, wo die jeweiligen Voxel der ersten dreidimensionalen MRT-Aufnahme die entsprechenden Voxel der zweiten dreidimensionalen MRT-Aufnahme ersetzen. Gegebenenfalls muss eine Bewegungskorrektur vorgenommen werden. Da die Voxel, die Blutgefäße repräsentieren, auf den Leber-Tonwert gesetzt sind, wird der Kontrastunterschied zwischen den Bereichen der dritten MRT-Aufnahme, die Blutgefäße darstellen, und den Bereichen der dritten MRT-Aufnahme, die gesunde Leberzellen darstellen, nivelliert.

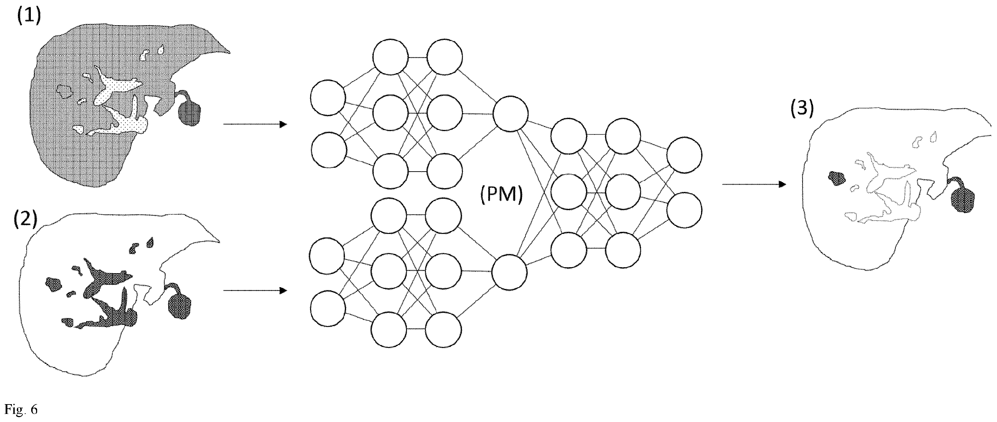

Figur 6 zeigt beispielhaft und schematisch eine weitere Ausführungsform der vorliegenden Erfindung (Ausführungsform C). Es wird eine erste MRT-Aufnahme (1) bereitgestellt, wobei die erste MRT-Aufnahme eine Leber oder einen Teil einer Leber eines Untersuchungsobjekts zeigt, wobei Blutgefäße in der Leber durch ein Kontrastmittel kontrastverstärkt (signalverstärkt) dargestellt sind.

Es wird eine zweite MRT-Aufnahme (2) bereitgestellt, wobei die zweite MRT-Aufnahme dieselbe Leber oder denselben Teil der Leber zeigt wie die erste MRT-Aufnahme, wobei das gesunde Lebergewebe (Parenchym) durch ein Kontrastmittel kontrastverstärkt (signalverstärkt) dargestellt ist.

Die erste MRT-Aufnahme (1) und die zweite MRT-Aufnahme (2) werden einem Vorhersagemodell (PM) zugeführt.

Das Vorhersagemodell (PM) ist konfiguriert, auf Basis der ersten MRT-Aufnahme (1) und der zweiten MRT-Aufnahme (2) eine dritte MRT-Aufnahme (3) zu erzeugen, wobei bei der dritten MRT-Aufnahme der Kontrastunterschied zwischen Strukturen, die auf Blutgefäße zurückzuführen sind und Strukturen, die auf gesunde Leberzellen zurückzuführen sind, nivelliert ist.

Das Vorhersagemodell wurde vorzugsweise mit Hilfe eines selbstlernenden Algorithmus in einem überwachten maschinellen Lernen mit einem Trainingsdatensatz erstellt. Der Trainingsdatensatz umfasst eine Vielzahl an ersten MRT-Aufnahmen, zweiten MRT-Aufnahmen und den dazugehörigen dritten MRT-Aufnahmen, wobei jede dritte MRT-Aufnahme eine Kombination einer ersten und einer zweiten MRT-Aufnahme ist, wobei in der dritten MRT-Aufnahme der Kontrastunterschied zwischen Strukturen, die auf Blutgefäße zurückzuführen sind, und Strukturen, die auf gesunde Leberzellen zurückzuführen sind, nivelliert ist. Der Trainingsdatensatz lässt sich beispielsweise mit Hilfe der Ausführungsformen A und/oder B der vorliegenden Erfindung generieren. Die Trainingsdaten werden in dieser Beschreibung auch als Referenz-MRT-Aufnahmen bezeichnet. Eine erste Referenz-MRT-Aufnahme ist dementsprechend eine MRT-Aufnahme, die eine Leber oder einen Teil einer Leber eines Untersuchungsobjekts zeigt, wobei Blutgefäße in der Leber durch ein Kontrastmittel kontrastverstärkt dargestellt sind; eine zweite Referenz-MRT-Aufnahme ist dementsprechend eine MRT-Aufnahme, die dieselbe Leber oder denselben Teil der Leber zeigt wie die erste Referenz-MRT-Aufnahme, wobei das gesunde Lebergewebe (Parenchym) durch ein Kontrastmittel kontrastverstärkt dargestellt ist; eine dritte Referenz-MRT-Aufnahme ist demensprechend eine erfindungsgemäße Kombination einer ersten und einer zweiten Referenz-MRT-Aufnahme, bei der der Kontrastunterschied zwischen Strukturen, die auf Blutgefäße zurückzuführen sind und Strukturen, die auf gesunde Leberzellen zurückzuführen sind, nivelliert ist.

Der selbstlernende Algorithmus erzeugt beim maschinellen Lernen ein statistisches Modell, das auf den Trainingsdaten beruht. Das heißt, es werden nicht einfach die Beispiele auswendig gelernt, sondern der Algorithmus "erkennt" Muster und Gesetzmäßigkeiten in den Trainingsdaten. So kann der Algorithmus auch unbekannte Daten beurteilen. Validierungsdaten können verwendet werden, um die Güte der Beurteilung unbekannter Daten zu prüfen.

Das Trainieren des selbstlernenden Algorithmus erfolgt mittels überwachten Lernens (engl.: *supervised learning*), d.h. dem Algorithmus werden erste und zweite Referenz-MRT-Aufnahmen präsentiert und es wird ihm mitgeteilt, welche dritten Referenz-MRT-Aufnahmen mit den jeweiligen ersten und zweiten Referenz-MRT-Aufnahmen verbunden sind. Der Algorithmus lernt dann eine Beziehung zwischen den Referenz-MRT-Aufnahmen, um für unbekannte erste und zweite MRT-Aufnahmen dritte MRT-Aufnahmen vorherzusagen (zu berechnen).

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545, WO2018/183044A1, WO2018/200493, WO2019/074938A1, WO2019/204406A1, WO2019/241659A1).

Vorzugsweise handelt es sich bei dem Vorhersagemodell um ein künstliches neuronales Netz.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der MRT-Aufnahmen herrschten) vorhanden sein.

In einem solchen Netzwerk dienen die Ausgangsneuronen dazu, für eine erste und eine zweite MRT-Aufnahme eine dritte MRT-Aufnahme.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufnahme zu erkennen. Das gleiche Objekt an einer anderen Position in der MRT-Aufnahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung zwischen den ersten und zweiten Referenz-MRT-Aufnahmen und den dritten Referenz-MRT-Aufnahmen, die verwendet werden können, um eine oder mehrere dritte MRT-Aufnahmen auf Basis von neuen ersten und zweiten MRT-Aufnahmen vorherzusagen.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um zu überprüfen, mit welcher Vorhersagegenauigkeit sich das trainierte Netzwerk auf unbekannte MRT-Aufnahmen anwenden lässt.

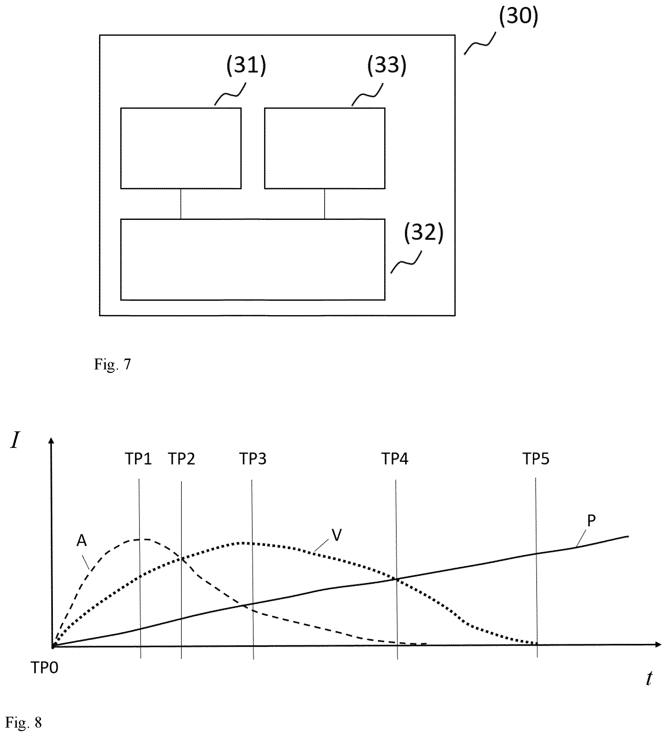

Figur 7 zeigt schematisch eine bevorzugte Ausführungsform des erfindungsgemäßen Systems. Das System (30) umfasst eine Empfangseinheit (31), eine Steuer- und Recheneinheit (32) und eine Ausgabeeinheit (33).

Die Steuer- und Recheneinheit (32) ist konfiguriert, die Empfangseinheit (32) zu veranlassen, mindestens eine erste MRT-Aufnahme eines Untersuchungsobjekts zu empfangen, wobei die mindestens eine erste MRT-Aufnahme eine Leber oder einen Teil einer Leber des Untersuchungsobjekts zeigt, wobei Blutgefäße in der Leber durch ein Kontrastmittel kontrastverstärkt dargestellt sind.

Die Steuer- und Recheneinheit (32) ist ferner konfiguriert, die Empfangseinheit (32) zu veranlassen, mindestens eine zweite MRT-Aufnahme eines Untersuchungsobjekts zu empfangen, wobei die mindestens eine zweite MRT-Aufnahme dieselbe Leber oder denselben Teil der Leber in der hepatobiliären Phase zeigt, wobei gesunde Leberzellen durch ein Kontrastmittel kontrastverstärkt dargestellt sind.

Die Steuer- und Recheneinheit (32) ist ferner konfiguriert, mindestens eine dritte MRT-Aufnahme durch Kombination der mindestens einen ersten MRT-Aufnahme und der mindestens einen zweiten MRT-Aufnahme und Nivellieren des Kontrastunterschieds zwischen Blutgefäßen und gesunden Leberzellen zu erzeugen.

Die Steuer- und Recheneinheit (32) ist ferner konfiguriert, die Ausgabeeinheit zu veranlassen, die mindestens eine dritte MRT-Aufnahme anzuzeigen, auszugeben oder in einem Datenspeicher zu speichern.

Figur 8 zeigt schematisch den zeitlichen Verlauf der Konzentrationen von Kontrastmittel in den Leberarterien (A), den Lebervenen (V) und den gesunden Leberzellen (P) nach einer intravenösen Applikation eines hepatobiliären Kontrastmittels als Bolus. Die Konzentrationen sind in Form der Signalintensitäten I in den genannten Arealen (Leberarterien, Lebervenen, Leberzellen) bei der Magnetresonanzmessung als Funktion der Zeit t dargestellt. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (V) steigt langsamer an als in den Leberarterien und erreicht ihr Maximum später (gepunktete Kurve). Die Konzentration des Kontrastmittels in den gesunden Leberzellen (P) steigt langsam an (durchgezogene Kurve) und erreicht ihr Maximum erst zu einem sehr viel späteren Zeitpunkt (das Maximum ist in der Figur 8 nicht dargestellt). Es lassen sich einige charakteristische Zeitpunkte definieren: Zum Zeitpunkt TP0 wird Kontrastmittel intravenös als Bolus appliziert. Zum Zeitpunkt TP 1 erreicht die Konzentration (die Signalintensität) des Kontrastmittels in den Leberarterien ihr Maximum. Zum Zeitpunkt TP2 schneiden sich die Kurven der Signalintensitäten bei den Leberarterien und den Lebervenen. Zum Zeitpunkt TP3 durchläuft die Konzentration (die Signalintensität) des Kontrastmittels in den Lebervenen ihr Maximum. Zum Zeitpunkt TP4 schneiden sich die Kurven der Signalintensitäten bei den Leberarterien und den Leberzellen. Zum Zeitpunkt T5 sind die Konzentrationen in den Leberarterien und den Lebervenen auf ein Niveau gesunken, bei dem sie keine messbare Kontrastverstärkung mehr verursachen.

In einer bevorzugten Ausführungsform handelt es sich bei der mindestens einen ersten MRT-Aufnahme um eine MRT-Aufnahme, die in der Zeitspanne zwischen TP0 und TP5, vorzugsweise zwischen TP0 und TP4 erzeugt worden ist. In einer bevorzugten Ausführungsform werden mindestens zwei erste MRT-Aufnahmen zur Berechnung der mindestens einen dritten MRT-Aufnahme verwendet, vorzugsweise eine MRT-Aufnahme, die in der Zeitspanne zwischen TP0 und TP3 erzeugt worden ist, und eine weitere MRT-Aufnahme, die in der Zeitspanne zwischen TP2 und TP4 erzeugt worden ist.

In einer bevorzugten Ausführungsform handelt es sich bei der mindestens einen zweiten MRT-Aufnahme um eine MRT-Aufnahme, die nach TP5 erzeugt worden ist.

## Patentansprüche

1. Computer-implementiertes Verfahren umfassend die Schritte
- Empfangen mindestens einer ersten MRT-Aufnahme (1) eines Untersuchungsobjekts, wobei die mindestens eine erste MRT-Aufnahme (1) eine Leber oder einen Teil einer Leber des Untersuchungsobjekts zeigt, wobei Blutgefäße (A, V) in der Leber durch ein hepatobiliäres Kontrastmittel kontrastverstärkt dargestellt sind,
- Empfangen mindestens einer zweiten MRT-Aufnahme (2) desselben Untersuchungsobjekts, wobei die mindestens eine zweite MRT-Aufnahme (2) dieselbe Leber oder denselben Teil der Leber in der hepatobiliären Phase zeigt, wobei gesunde Leberzellen (P) durch das hepatobiliäre Kontrastmittel kontrastverstärkt dargestellt sind,
**dadurch gekennzeichnet, dass** das Verfahren ferner die folgenden Schritte umfasst:
- Erzeugen mindestens einer dritten MRT-Aufnahme (3) durch Kombinieren der mindestens einen ersten MRT-Aufnahme (1) mit der mindestens einen zweiten MRT-Aufnahme (2) und Nivellieren des Kontrastunterschieds zwischen den in der mindestens einen ersten MRT-Aufnahme (1) kontrastverstärkt dargestellten Blutgefäßen (A, V) und den in der mindestens einen zweiten MRT-Aufnahme (2) kontrastverstärkt dargestellten gesunden Leberzellen (P),
- Anzeigen und/oder Ausgeben der mindestens einen dritten MRT-Aufnahme (3) und/oder Speichern der mindestens einen dritten MRT-Aufnahme (3) in einem Datenspeicher.

2. Verfahren gemäß Anspruch 1, wobei es sich bei der mindestens einen ersten MRT-Aufnahme (1) um eine T1-gewichtete Darstellung der Leber oder des Teils der Leber in einer venösen Phase nach Applikation eines hepatobiliären, paramagnetischen Kontrastmittels handelt.

3. Verfahren gemäß Anspruch 1 oder 2, wobei es sich bei der mindestens einen zweiten MRT-Aufnahme (2) um eine T1-gewichtete Darstellung der Leber oder des Teils der Leber in der hepatobiliären Phase nach Applikation eines hepatobiliären, paramagnetischen Kontrastmittels handelt.

4. Verfahren gemäß einem der Ansprüche 1 bis 3, wobei es sich bei dem Kontrastmittel um einen Stoff oder ein Stoffgemisch mit der Gadoxetsäure oder einem Salz der Gadoxetsäure als kontrastverstärkenden Wirkstoff, bevorzugt um Gd-EOB-DTPA Dinatrium handelt.

5. Verfahren gemäß einem der Ansprüche 1 bis 4, wobei der Schritt Erzeugen mindestens einer dritten MRT-Aufnahme (3) die folgenden Teilschritte umfasst:
- Identifizieren von Bereichen in der mindestens einen ersten MRT-Aufnahme (1), die Blutgefäße (A, V) darstellen, durch Identifizieren derjenigen Pixel oder Voxel der mindestens einen ersten MRT-Aufnahme (1), die einen Tonwert aufweisen, der innerhalb eines ersten definierten Tonwert-Bandes liegt, und/oder
- Identifizieren von Bereichen in der mindestens einen zweiten MRT-Aufnahme (2), die gesunde Leberzellen (P) darstellen, durch Identifizieren derjenigen Pixel oder Voxel der mindestens einen zweiten MRT-Aufnahme (2), die einen Tonwert aufweisen, der innerhalb eines zweiten definierten Tonwert-Bandes liegt, und
- zumindest partielles Übereinanderlegen der mindestens einen ersten MRT-Aufnahme (1) und der mindestens einen zweiten MRT-Aufnahme (2) und dabei Erzeugen der mindestens einen dritten MRT-Aufnahme (3), wobei die Tonwerte der Pixel oder Voxel mit dem Tonwert innerhalb der ersten definierten Bandbreite und der Pixel oder Voxel mit dem Tonwert innerhalb der zweiten definierten Bandbreite in der mindestens einen dritten MRT-Aufnahme (3) auf einen gemeinsamen Tonwert gesetzt werden.

6. Verfahren gemäß einem der Ansprüche 1 bis 4, wobei der Schritt Erzeugen mindestens einer dritten MRT-Aufnahme (3) die folgenden Teilschritte umfasst:
- Identifizieren der Blutgefäße (A, V) in der mindestens einen ersten MRT-Aufnahme (1) mittels eines Segmentierverfahrens,
- Identifizieren derjenigen Strukturen in der mindestens einen zweiten MRT-Aufnahme (2), die den Blutgefäßen (A, V) in der mindestens einen ersten MRT-Aufnahme (1) entsprechen,
- Identifizieren eines Tonwerts von gesunden Leberzellen (P) in der mindestens einen zweiten MRT-Aufnahme (2),
- Setzen der Tonwerte der Strukturen in der mindestens einen zweiten MRT-Aufnahme (2), die den Blutgefäßen (A, V) in der mindestens einen ersten MRT-Aufnahme (1) entsprechen, auf den Tonwert von gesunden Leberzellen (P).

7. Verfahren gemäß einem der Ansprüche 1 bis 4, wobei der Schritt Erzeugen mindestens einer dritten MRT-Aufnahme (3) die folgenden Teilschritte umfasst:
- Zuführen der mindestens einen ersten MRT-Aufnahme (1) und der mindestens einen zweiten MRT-Aufnahme (2) zu einem Vorhersagemodell (PM), wobei das Vorhersagemodell (PM) mittels überwachten Lernens anhand von Referenz-MRT-Aufnahmen trainiert worden ist, aus mindestens einer ersten Referenz-MRT-Aufnahme und mindestens einer zweiten Referenz-MRT-Aufnahme mindestens eine dritte Referenz-MRT-Aufnahme zu erzeugen, wobei bei der mindestens einen dritten Referenz-MRT-Aufnahme der Kontrastunterschied zwischen Strukturen, die auf Blutgefäße (A, V) zurückzuführen sind und Strukturen, die auf gesunde Leberzellen (P) zurückzuführen sind, nivelliert ist,
- Empfangen mindestens einer dritten MRT-Aufnahme (3) als Ausgabe von dem Vorhersagemodell (PM).

8. Verfahren gemäß Anspruch 7, wobei es sich bei dem Vorhersagemodell (PM) um ein künstliches neuronales Netz handelt.

9. System (30) umfassend
• eine Empfangseinheit (31),
• eine Steuer- und Recheneinheit (32) und
• eine Ausgabeeinheit (33),
- wobei die Steuer- und Recheneinheit (32) konfiguriert ist, die Empfangseinheit (31) zu veranlassen, mindestens eine erste MRT-Aufnahme (1) eines Untersuchungsobjekts zu empfangen, wobei die mindestens eine erste MRT-Aufnahme (1) eine Leber oder einen Teil einer Leber des Untersuchungsobjekts zeigt, wobei Blutgefäße (A, V) in der Leber durch ein hepatobiliäres Kontrastmittel kontrastverstärkt dargestellt sind,
- wobei die Steuer- und Recheneinheit (32) konfiguriert ist, die Empfangseinheit (31) zu veranlassen, mindestens eine zweite MRT-Aufnahme (2) eines Untersuchungsobjekts zu empfangen, wobei die mindestens eine zweite MRT-Aufnahme (2) dieselbe Leber oder denselben Teil der Leber in der hepatobiliären Phase zeigt, wobei gesunde Leberzellen (P) durch das hepatobiliäre Kontrastmittel kontrastverstärkt dargestellt sind,
**dadurch gekennzeichnet, dass**
- die Steuer- und Recheneinheit (32) konfiguriert ist,
∘ mindestens eine dritte MRT-Aufnahme (3) durch Kombination der mindestens einen ersten MRT-Aufnahme (1) und der mindestens einen zweiten MRT-Aufnahme (2) und Nivellieren des Kontrastunterschieds zwischen den in der mindestens einen ersten MRT-Aufnahme (1) kontrastverstärkt dargestellten Blutgefäßen (A, V) und den in der mindestens einen zweiten MRT-Aufnahme (2) kontrastverstärkt dargestellten gesunden Leberzellen (P) zu erzeugen, und
∘ die Ausgabeeinheit (33) zu veranlassen, die mindestens eine dritte MRT-Aufnahme (3) anzuzeigen, auszugeben oder in einem Datenspeicher zu speichern.

10. Computerprogrammprodukt umfassend ein Computerprogramm, das in einen Arbeitsspeicher eines Computersystems geladen werden kann und dort das Computersystem dazu veranlasst, folgende Schritte auszuführen:
- Empfangen mindestens einer ersten MRT-Aufnahme (1) eines Untersuchungsobjekts, wobei die mindestens eine erste MRT-Aufnahme (1) eine Leber oder einen Teil einer Leber des Untersuchungsobjekts zeigt, wobei Blutgefäße (A, V) in der Leber durch ein hepatobiliäres Kontrastmittel kontrastverstärkt dargestellt sind,
- Empfangen mindestens einer zweiten MRT-Aufnahme (2) desselben Untersuchungsobjekts, wobei die mindestens eine zweite MRT-Aufnahme (2) dieselbe Leber oder denselben Teil der Leber in der hepatobiliären Phase zeigt, wobei gesunde Leberzellen (P) durch das hepatobiliäre Kontrastmittel kontrastverstärkt dargestellt sind,
**dadurch gekennzeichnet, dass** das Computerprogramm das Computersystem ferner veranlasst, die folgenden Schritte auszuführen:
- Erzeugen mindestens einer dritten MRT-Aufnahme (3) durch Kombinieren der mindestens einen ersten MRT-Aufnahme (1) mit der mindestens einen zweiten MRT-Aufnahme (2) und Nivellieren des Kontrastunterschieds zwischen den in der mindestens einen ersten MRT-Aufnahme (1) kontrastverstärkt dargestellten Blutgefäßen (A, V) und den in der mindestens einen zweiten MRT-Aufnahme (2) kontrastverstärkt dargestellten gesunden Leberzellen (P),
- Anzeigen und/oder Ausgeben der mindestens einen dritten MRT-Aufnahme (3) und/oder Speichern der mindestens einen dritten MRT-Aufnahme (3) in einem Datenspeicher.

11. Computerprogrammprodukt gemäß Anspruch 10, wobei das Computerprogramm, das Computersystem dazu veranlasst, einen oder mehrere der in den Ansprüchen 2 bis 8 aufgeführten Schritte auszuführen.

12. Verwendung eines hepatobiliären Kontrastmittels in einem MRT-Verfahren, wobei das MRT-Verfahren die folgenden Schritte umfasst:
- Applizieren des Kontrastmittels, wobei sich das Kontrastmittel in einer Leber eines Untersuchungsobjekts verteilt,
- Erzeugen mindestens einer ersten MRT-Aufnahme (1), wobei die mindestens eine erste MRT-Aufnahme (1) die Leber oder einen Teil der Leber des Untersuchungsobjekts zeigt, wobei Blutgefäße (A, V) in der Leber durch das Kontrastmittel kontrastverstärkt dargestellt sind,
- Erzeugen mindestens einer zweiten MRT-Aufnahme (2), wobei die mindestens eine zweite MRT-Aufnahme (2) dieselbe Leber oder denselben Teil der Leber in der hepatobiliären Phase zeigt, wobei gesunde Leberzellen (P) durch das Kontrastmittel kontrastverstärkt dargestellt sind,
**dadurch gekennzeichnet, dass** das MRT-Verfahren ferner die folgenden Schritte umfasst:
- Erzeugen mindestens einer dritten MRT-Aufnahme (3) durch Kombinieren der mindestens einen ersten MRT-Aufnahme (1) mit der mindestens einen zweiten MRT-Aufnahme (2) und Nivellieren des Kontrastunterschieds zwischen den in der mindestens einen ersten MRT-Aufnahme (1) kontrastverstärkt dargestellten Blutgefäßen (A, V) und den in der mindestens einen zweiten MRT-Aufnahme (2) kontrastverstärkt dargestellten gesunden Leberzellen (P),
- Anzeigen und/oder Ausgeben der mindestens einen dritten MRT-Aufnahme (3) und/oder Speichern der mindestens einen dritten MRT-Aufnahme (3) in einem Datenspeicher.

13. Kit umfassend ein hepatobiliäres MRT-Kontrastmittel und ein Computerprogrammprodukt gemäß Anspruch 10 oder 11.

14. Kit gemäß Anspruch 13, wobei das MRT-Kontrastmittel ein Stoff oder ein Stoffgemisch mit Gadoxetsäure oder einem Salz der Gadoxetsäure als kontrastverstärkenden Wirkstoff ist, bevorzugt Gd-EOB-DTPA Dinatrium.

## Claims

1. Computer-implemented method comprising the steps of
- receiving at least one first MRI image (1) of an examination object, the at least one first MRI image (1) showing a liver or a portion of a liver of the examination object, blood vessels (A, V) in the liver being depicted with contrast enhancement as a result of a hepatobiliary contrast agent,
- receiving at least one second MRI image (2) of the same examination object, the at least one second MRI image (2) showing the same liver or the same portion of the liver in the hepatobiliary phase, healthy liver cells (P) being depicted with contrast enhancement as a result of the hepatobiliary contrast agent,
**characterized in that** the method further comprises the following steps:
- generating at least one third MRI image (3) by combining the at least one first MRI image (1) with the at least one second MRI image (2) and levelling out the difference in contrast between the blood vessels (A, V) depicted with contrast enhancement in the at least one first MRI image (1) and the healthy liver cells (P) depicted with contrast enhancement in the at least one second MRI image (2),
- displaying and/or outputting the at least one third MRI image (3) and/or storing the at least one third MRI image (3) in a data memory.

2. Method according to Claim 1, wherein the at least one first MRI image (1) is a T1-weighted depiction of the liver or the portion of the liver in a venous phase after administration of a hepatobiliary, paramagnetic contrast agent.

3. Method according to Claim 1 or 2, wherein the at least one second MRI image (2) is a T1-weighted depiction of the liver or the portion of the liver in the hepatobiliary phase after administration of a hepatobiliary, paramagnetic contrast agent.

4. Method according to any of Claims 1 to 3, wherein the contrast agent is a substance or a substance mixture with gadoxetic acid or a gadoxetic acid salt as contrast-enhancing active substance, preferably Gd-EOB-DTPA disodium.

5. Method according to any of Claims 1 to 4, wherein the step for generating at least one third MRI image (3) comprises the following sub-steps:
- identifying regions in the at least one first MRI image (1) that depict blood vessels (A, V) by identifying those pixels or voxels of the at least one first MRI image (1) that have a tonal value within a first defined tonal-value band, and/or
- identifying regions in the at least one second MRI image (2) that depict healthy liver cells (P) by identifying those pixels or voxels of the at least one second MRI image (2) that have a tonal value within a second defined tonal-value band, and
- at least partially superposing the at least one first MRI image (1) and the at least one second MRI image (2) and, in doing so, generating the at least one third MRI image (3), the tonal values of the pixels or voxels having the tonal value within the first defined range and of the pixels or voxels having the tonal value within the second defined range being set to a common tonal value in the at least one third MRI image (3).

6. Method according to any of Claims 1 to 4, wherein the step for generating at least one third MRI image (3) comprises the following sub-steps:
- identifying the blood vessels (A, V) in the at least one first MRI image (1) by means of a segmentation method,
- identifying those structures in the at least one second MRI image (2) that correspond to the blood vessels (A, V) in the at least one first MRI image (1),
- identifying a tonal value of healthy liver cells (P) in the at least one second MRI image (2),
- setting the tonal values of the structures in the at least one second MRI image (2) that correspond to the blood vessels (A, V) in the at least one first MRI image (1) to the tonal value of healthy liver cells (P).

7. Method according to any of Claims 1 to 4, wherein the step for generating at least one third MRI image (3) comprises the following sub-steps:
- feeding the at least one first MRI image (1) and the at least one second MRI image (2) to a prediction model (PM), the prediction model (PM) having been trained by means of supervised learning on the basis of reference MRI images to generate at least one third reference MRI image from at least one first reference MRI image and at least one second reference MRI image, the difference in contrast between structures attributable to blood vessels (A, V) and structures attributable to healthy liver cells (P) being levelled out in the at least one third reference MRI image,
- receiving at least one third MRI image (3) as output from the prediction model (PM).

8. Method according to Claim 7, wherein the prediction model (PM) is an artificial neural network.

9. System (30) comprising
• a receiving unit (31),
• a control and calculation unit (32) and
• an output unit (33),
- the control and calculation unit (32) being configured to cause the receiving unit (31) to receive at least one first MRI image (1) of an examination object, the at least one first MRI image (1) showing a liver or a portion of a liver of the examination object, blood vessels (A, V) in the liver being depicted with contrast enhancement as a result of a hepatobiliary contrast agent,
- the control and calculation unit (32) being configured to cause the receiving unit (31) to receive at least one second MRI image (2) of an examination object, the at least one second MRI image (2) showing the same liver or the same portion of the liver in the hepatobiliary phase, healthy liver cells (P) being depicted with contrast enhancement as a result of the hepatobiliary contrast agent,
**characterized in that**
- the control and calculation unit (32) is configured
∘ to generate at least one third MRI image (3) by combining the at least one first MRI image (1) and the at least one second MRI image (2) and levelling out the difference in contrast between the blood vessels (A, V) depicted with contrast enhancement in the at least one first MRI image (1) and the healthy liver cells (P) depicted with contrast enhancement in the at least one second MRI image (2), and
∘ to cause the output unit (33) to display the at least one third MRI image (3), to output it or to store it in a data memory.

10. Computer program product comprising a computer program which can be loaded into a working memory of a computer system, where it causes the computer system to execute the following steps:
- receiving at least one first MRI image (1) of an examination object, the at least one first MRI image (1) showing a liver or a portion of a liver of the examination object, blood vessels (A, V) in the liver being depicted with contrast enhancement as a result of a hepatobiliary contrast agent,
- receiving at least one second MRI image (2) of the same examination object, the at least one second MRI image (2) showing the same liver or the same portion of the liver in the hepatobiliary phase, healthy liver cells (P) being depicted with contrast enhancement as a result of the hepatobiliary contrast agent,
**characterized in that** the computer program further causes the computer system to execute the following steps:
- generating at least one third MRI image (3) by combining the at least one first MRI image (1) with the at least one second MRI image (2) and levelling out the difference in contrast between the blood vessels (A, V) depicted with contrast enhancement in the at least one first MRI image (1) and the healthy liver cells (P) depicted with contrast enhancement in the at least one second MRI image (2),
- displaying and/or outputting the at least one third MRI image (3) and/or storing the at least one third MRI image (3) in a data memory.

11. Computer program product according to Claim 10, wherein the computer program causes the computer system to execute one or more of the steps listed in Claims 2 to 8.

12. Use of a hepatobiliary contrast agent in an MRI method, the MRI method comprising the following steps:
- administering the contrast agent, the contrast agent spreading in a liver of an examination object,
- generating at least one first MRI image (1), the at least one first MRI image (1) showing the liver or a portion of a liver of the examination object, blood vessels (A, V) in the liver being depicted with contrast enhancement as a result of the contrast agent,
- generating at least one second MRI image (2), the at least one second MRI image (2) showing the same liver or the same portion of the liver in the hepatobiliary phase, healthy liver cells (P) being depicted with contrast enhancement as a result of the contrast agent, **characterized in that** the MRI method further comprises the following steps:
- generating at least one third MRI image (3) by combining the at least one first MRI image (1) with the at least one second MRI image (2) and levelling out the difference in contrast between the blood vessels (A, V) depicted with contrast enhancement in the at least one first MRI image (1) and the healthy liver cells (P) depicted with contrast enhancement in the at least one second MRI image (2),
- displaying and/or outputting the at least one third MRI image (3) and/or storing the at least one third MRI image (3) in a data memory.

13. Kit comprising a hepatobiliary MRI contrast agent and a computer program product according to Claim 10 or 11.

14. Kit according to Claim 13, wherein the MRI contrast agent is a substance or a substance mixture with gadoxetic acid or a gadoxetic acid salt as contrast-enhancing active substance, preferably Gd-EOB-DTPA disodium.

## Revendications

1. Procédé mis en œuvre par ordinateur, comprenant les étapes suivantes
- recevoir au moins une première image IRM (1) d'un objet à examiner, ladite au moins une première image IRM (1) montrant un foie ou une partie d'un foie du objet à examiner, des vaisseaux sanguins (A, V) du foie étant représentés avec un contraste renforcé par un agent de contraste hépatobiliaire,
- recevoir au moins une deuxième image IRM (2) du même objet à examiner, ladite au moins une deuxième image IRM (2) montrant le même foie ou la même partie du foie dans la phase hépatobiliaire, des cellules hépatiques saines (P) étant représentées avec un contraste renforcé par l'agent de contraste hépatobiliaire,
**caractérisé en ce que** le procédé comprend en outre les étapes suivantes :
- générer au moins une troisième image IRM (3) en combinant ladite au moins une première image IRM (1) avec ladite au moins une deuxième image IRM (2) et en nivelant la différence de contraste entre les vaisseaux sanguins (A, V) représentés avec un contraste renforcé dans ladite au moins une première image IRM (1) et les cellules hépatiques saines (P) représentées avec un contraste renforcé dans ladite au moins une deuxième image IRM (2),
- afficher et/ou fournir en sortie ladite au moins une troisième image IRM (3) et/ou mémoriser ladite au moins une troisième image IRM (3) dans une mémoire de données.

2. Procédé selon la revendication 1, dans lequel ladite au moins une première image IRM (1) est une représentation pondérée en T1 du foie ou de la partie du foie dans une phase veineuse après application d'un agent de contraste paramagnétique hépatobiliaire.

3. Procédé selon la revendication 1 ou 2, dans lequel ladite au moins une deuxième image IRM (2) est une représentation pondérée en T1 du foie ou de la partie du foie dans la phase veineuse après application d'un agent de contraste paramagnétique hépatobiliaire.

4. Procédé selon l'une des revendications 1 à 3, dans lequel l'agent de contraste est une substance ou un mélange de substances contenant de l'acide gadoxétique ou un sel de l'acide gadoxétique en tant qu'agent de renforcement du contraste, de préférence du Gd-EOB-DTPA disodique.

5. Procédé selon l'une des revendications 1 à 4, dans lequel l'étape de génération d'au moins une troisième image IRM (3) comprend les sous-étapes suivantes :
- identifier des zones dans ladite au moins une première image IRM (1) qui représentent des vaisseaux sanguins (A, V) en identifiant les pixels ou voxels de ladite au moins une première image IRM (1) qui présentent une valeur tonale se situant à l'intérieur d'une première bande de valeurs tonales définie, et/ou
- identifier des zones dans ladite au moins une deuxième image IRM (2) qui représentent des cellules hépatiques saines (P) en identifiant les pixels ou voxels de ladite au moins une deuxième image IRM (2) qui présentent une valeur tonale se situant à l'intérieur d'une deuxième bande de valeurs tonales définie, et
- superposer au moins partiellement ladite au moins une première image IRM (1) et ladite au moins une deuxième image IRM (2) et ainsi, générer ladite au moins une troisième image IRM (3), les valeurs tonales des pixels ou voxels dont la valeur tonale se situe à l'intérieur de la première largeur de bande définie et des pixels ou voxels dont la valeur tonale se situe à l'intérieur de la deuxième largeur de bande définie étant réglées à une valeur tonale commune dans ladite au moins une troisième image IRM (3).

6. Procédé selon l'une des revendications 1 à 4, dans lequel l'étape de génération d'au moins une troisième image IRM (3) comprend les sous-étapes suivantes :
- identifier les vaisseaux sanguins (A, V) dans ladite au moins une première image IRM (1) au moyen d'un procédé de segmentation,
- identifier les structures dans ladite au moins une deuxième image IRM (2) qui correspondent aux vaisseaux sanguins (A, V) dans ladite au moins une première image IRM (1),
- identifier une valeur tonale de cellules hépatiques saines (P) dans ladite au moins une deuxième image IRM (2),
- régler les valeurs tonales des structures dans ladite au moins une deuxième image IRM (2) qui correspondent aux vaisseaux sanguins (A, V) dans ladite au moins une première image IRM (1) à la valeur tonale des cellules hépatiques saines (P).

7. Procédé selon l'une des revendications 1 à 4, dans lequel l'étape de génération d'au moins une troisième image IRM (3) comprend les sous-étapes suivantes :
- fournir ladite au moins une première image IRM (1) et ladite au moins une deuxième image IRM (2) à un modèle de prédiction (PM), le modèle de prédiction (PM) ayant été entraîné par apprentissage supervisé sur la base d'images IRM de référence, pour générer au moins une troisième image IRM de référence à partir d'au moins une première image IRM de référence et d'au moins une deuxième image IRM de référence, la différence de contraste entre des structures attribuables aux vaisseaux sanguins (A, V) et des structures attribuables aux cellules hépatiques saines (P) étant nivelée dans ladite au moins une troisième image IRM de référence,
- recevoir au moins une troisième image IRM (3) en tant que sortie du modèle de prédiction (PM).

8. Procédé selon la revendication 7, dans lequel le modèle de prédiction (PM) est un réseau neuronal artificiel.

9. Système (30) comprenant
• une unité de réception (31),
• une unité de commande et de calcul (32) et
• une unité de sortie (33),
- dans lequel l'unité de commande et de calcul (32) est configurée pour amener l'unité de réception (31) à recevoir au moins une première image IRM (1) d'un objet à examiner, ladite au moins une première image IRM (1) montrant un foie ou une partie d'un foie du objet à examiner, des vaisseaux sanguins (A, V) du foie étant représentés avec un contraste renforcé par un agent de contraste hépatobiliaire,
- dans lequel l'unité de commande et de calcul (32) est configurée pour amener l'unité de réception (31) à recevoir au moins une deuxième image IRM (2) d'un objet à examiner, ladite au moins une deuxième image IRM (2) montrant le même foie ou la même partie du foie dans la phase hépatobiliaire, des cellules hépatiques saines (P) étant représentées avec un contraste renforcé par l'agent de contraste hépatobiliaire,
**caractérisé en ce que**
- l'unité de commande et de calcul (32) est configurée pour
∘ générer au moins une troisième image IRM (3) en combinant ladite au moins une première image IRM (1) et ladite au moins une deuxième image IRM (2) et en nivelant la différence de contraste entre les vaisseaux sanguins (A, V) représentés avec un contraste renforcé dans ladite au moins une première image IRM (1) et les cellules hépatiques saines (P) représentées avec un contraste renforcé dans ladite au moins une deuxième image IRM (2),
o amener l'unité de sortie (33) à afficher, délivrer ou mémoriser dans une mémoire de données ladite au moins une troisième image IRM (3).

10. Produit programme d'ordinateur comprenant un programme d'ordinateur qui peut être chargé dans une mémoire vive d'un système d'ordinateur et qui, dans celui-ci, amène le système d'ordinateur à mettre en œuvre les étapes suivantes :
- recevoir au moins une première image IRM (1) d'un sujet à examiner, ladite au moins une première image IRM (1) montrant un foie ou une partie d'un foie du sujet à examiner, des vaisseaux sanguins (A, V) du foie étant représentés avec un contraste renforcé par un agent de contraste hépatobiliaire,
- recevoir au moins une deuxième image IRM (2) du même objet à examiner, ladite au moins une deuxième image IRM (2) montrant le même foie ou la même partie du foie dans la phase hépatobiliaire, des cellules hépatiques saines (P) étant représentées avec un contraste renforcé par l'agent de contraste hépatobiliaire,
**caractérisé en ce que** le programme d'ordinateur amène en outre le système d'ordinateur à effectuer les étapes suivantes :
- générer au moins une troisième image IRM (3) en combinant ladite au moins une première image IRM (1) avec ladite au moins une deuxième image IRM (2) et en nivelant la différence de contraste entre les vaisseaux sanguins (A, V) représentés avec un contraste renforcé dans ladite au moins une première image IRM (1) et les cellules hépatiques saines (P) représentées avec un contraste renforcé dans ladite au moins une deuxième image IRM (2),
- afficher et/ou fournir en sortie ladite au moins une troisième image IRM (3) et/ou mémoriser ladite au moins une troisième image IRM (3) dans une mémoire de données.

11. Produit programme d'ordinateur selon la revendication 10, dans lequel le programme d'ordinateur amène le système d'ordinateur à mettre en œuvre une ou plusieurs des étapes mentionnées dans les revendications 2 à 8.

12. Utilisation d'un agent de contraste hépatobiliaire dans un procédé d'IRM, le procédé d'IRM comprenant les étapes suivantes :
- appliquer l'agent de contraste, l'agent de contraste se répartissant dans le foie d'un objet à examiner,
- générer au moins une première image IRM (1), ladite au moins une première image IRM (1) montrant le foie ou une partie du foie du objet à examiner, des vaisseaux sanguins (A, V) du foie étant représentés avec un contraste renforcé par l'agent de contraste,
- générer au moins une deuxième image IRM (2), ladite au moins une deuxième image IRM (2) montrant le même foie ou la même partie du foie dans la phase hépatobiliaire, des cellules hépatiques saines (P) étant représentées avec un contraste renforcé par l'agent de contraste,
**caractérisé en ce que** le procédé d'IRM comprend en outre les étapes suivantes :
- générer au moins une troisième image IRM (3) en combinant ladite au moins une première image IRM (1) avec ladite au moins une deuxième image IRM (2) et en nivelant la différence de contraste entre les vaisseaux sanguins (A, V) représentés avec un contraste renforcé dans ladite au moins une première image IRM (1) et les cellules hépatiques saines (P) représentées avec un contraste renforcé dans ladite au moins une deuxième image IRM (2),
- afficher et/ou fournir en sortie ladite au moins une troisième image IRM (3) et/ou mémoriser ladite au moins une troisième image IRM (3) dans une mémoire de données.

13. Kit comprenant un agent de contraste d'IRM hépatobiliaire et un produit programme d'ordinateur selon la revendication 10 ou 11.

14. Kit selon la revendication 13, dans lequel l'agent de contraste d'IRM est une substance ou un mélange de substances contenant de l'acide gadoxétique ou un sel de l'acide gadoxétique en tant qu'agent de renforcement du contraste, de préférence du Gd-EOB-DTPA disodique.
